# FASCICULE DE BREVET EUROPEEN

(11) **EP 0 611 225 B1**
(45) Date de publication et mention de la délivrance du brevet: **03.09.1997**
(21) Numéro de dépôt: 94420026.0
(22) Date de dépôt: 27.01.1994
(51) Int. Cl.: A61F 2/36

(54) **Tige fémorale pour prothèse de hanche**
Femurales Schaftteil einer Hüftgelenkprothese
Femoral stem for hip prothesis

(30) Priorité: 09.02.1993 FR 9301692
(43) Date de publication de la demande: 17.08.1994
(73) Titulaire: MEDINOV SA, F-42312 Roanne Cedex (FR)
(72) Inventeur: Colombier Michel, F-42312 Roanne (FR); Moyen Bernard, F-69003 Lyon (FR); Lerat Jean Luc, Hôpital E. Herriot, F-69437 Lyon (FR); Trouilloud Pierre, F-21000 Dijon (FR); Rebouillat Jacques,, F-83000 Toulon (FR); Grammont Paul,, F-21000 Dijon (FR)
(74) Mandataire: Thivillier, Patrick

(56) Documents cités:
- EP-A- 0 038 908
- EP-A- 0 307 646
- EP-A- 0 428 303
- FR-A- 2 647 669
- US-A- 4 546 501

## Description

L'invention concerne le secteur technique des implants orthopédiques, plus particulièrement les tiges fémorales de reprise.

Les cas pathologiques de reprise d'anciennes arthroplasties de la hanche au moyen de prothèses, sont de plus en plus fréquents. Ces derniers résultent le plus souvent du descellement des tiges fémorales, provoqué par exemple, par le vieillissement du ciment ou bien par des formes de tiges non congruentes ou bien encore, suite à des ruptures du fémur, au niveau de l'extrémité distale de la tige en cas de chutes.

Ces cas pathologiques génèrent soit une dégradation de la partie proximale du fémur, se traduisant généralement par un évasement résultant d'un amincissement des corticales, soit une rupture du fémur en partie médiane.

Il est donc nécessaire de remplacer la tige fémorale d'origine par un modèle de longueur plus importante, afin d'avoir un appui distal suffisant. Ces modèles constituent des tiges fémorales de reprise.

Par le document EP-A-0307646, on connaît une tige fémorale de reprise pour la hanche, comprenant un élément métaphysaire et un élément diaphysaire en deux parties distinctes et indépendantes avec des agencements d'accouplement aptes à rendre solidaire ces éléments.

Le problème que se propose de résoudre l'invention, est de réaliser une tige fémorale de reprise déterminée pour assurer une mise en compression mécanique de la partie proximale sur la partie distale, afin de solliciter fortement la corticale proximale détruite. Sous l'effort de compression, la corticale s'épaissie pour épouser la forme de l'implant souvent recouvert de couches ostéoconductices.

Un autre problème que se propose de résoudre l'invention est de supprimer l'appui distal, afin d'obtenir seulement un appui proximal correspondant au modèle biomécanique généralement recherché.

Pour résoudre ces différents problèmes, il convient de tenir compte également, de la double courbure anatomique du fémur dans le plan sagittal. Certaines prothèses de hanche, qui, cependant, ne constituent pas des implants de reprise, sont déterminées pour reproduire cette double courbure anatomique.

On peut citer par exemple, le document EP-A-0038908 qui définit une prothèse de hanche dont la tige courbe, dans le plan antéropostérieur, présente dans la région proximale, une courbure dont le centre est déplacé antérieurement et, dans la région distale, une courbure dont le centre est déplacé postérieurement. En faisant abstraction du fait de l'application différente de ce type de prothèse, cette dernière ne permet pas de résoudre le double problème posé précédemment, concernant la compression mécanique de la partie proximale sans appui distal.

Selon l'invention, pour résoudre ces différents problèmes, il a été conçu et mis au point une tige fémorale de reprise conforme aux caractéristiques de la revendication 1.

Il en résulte que la combinaison du blocage avec la présence des deux courbes de valeurs différentes, permet de créer un système auto-bloquant, de sorte que la partie osseuse proximale est mise en compression par rapport à la partie osseuse distale.

Pour résoudre le problème posé d'obtenir, avant introduction de la tige fémorale, le montage à libre rotation de l'élément métaphysaire par rapport à l'élément diaphysaire, les agencements sont constitués par un trou débouchant formé sur la totalité de la hauteur de l'élément métaphysaire, pour recevoir avec capacité de rotation, une portée cylindrique formée en bout de l'élément diaphysaire et dont l'extrémité est conformée pour coopérer avec au moins un moyen d'assemblage rapporté dans l'élément métaphysaire.

Avantageusement, les moyens sont constitués par un écrou logé dans un chambrage du trou débouchant de l'élément métaphysaire et coopérant avec une portée filetée formée en bout de la portée cylindrique.

La portée cylindrique, au niveau de son raccordement avec l'élément diaphysaire présente une portée tronconique coopérant avec une portée complémentaire du trou de l'élément métaphysaire.

Pour résoudre le problème posé de diminuer la rupture d'élasticité entre le métal et l'os, l'extrémité distale de l'élément diaphysaire présente une fente.

Dans une forme de réalisation préférée, l'élément diaphysaire est recouvert d'une matière céramique dense, non ostéoconductrice et à résorption programmable en modifiant la composition de ladite matière.

Pour résoudre le problème posé d'avoir une tige de reprise la plus congruente à l'os au niveau de la partie distale, elle présente plusieurs éléments diaphysaires composés d'une tige circulaire de différents diamètres et de différentes longueurs.

L'invention est exposée, ci-après plus en détail à l'aide des dessins annexés, dans lesquels :
La figure 1 est une vue en coupe longitudinale considérée dans le plan frontal, avant assemblage des éléments constitutifs de la tige fémorale selon l'invention.
La figure 2 est une vue de côté correspondant à la figure 1.
La figure 3 est une vue en coupe correspondant à la figure 1, après solidarisation des éléments métaphysaire et diaphysaire avant blocage de ces derniers.
La figure 4 est une vue de profil correspondant à la figure 3.
La figure 5 montre la mise en place de la tige fémorale dans le canal médullaire après blocage des éléments métaphysaire et diaphysaire en position de compression.

La tige fémorale de reprise comprend un élément métaphysaire (1) et un élément diaphysaire (2) en deux parties distinctes et indépendantes. Chaque élément (1) et (2) a une courbure sagittale antéro-postérieure spécifique (1a) et (2a) afin de correspondre à la double courbure anatomique du fémur. Ces courbures (1a) et (2a) ressortent par exemple de l'enseignement de la demande de brevet européen 91420329.4 (EP-A-0477113) dont la demanderesse est également titulaire.

A noter également que les éléments (1) et (2) présentent, dans le plan frontal, des rayons de courbure (1b) (2b).

Selon une caractéristique importante de l'invention, les deux éléments (1) et (2) sont préalablement assemblés avant leur introduction dans le canal médullaire du fémur, afin d'être solidarisés avec capacité de libre rotation.

Dans ce but, et dans la forme de réalisation illustrée aux figures des dessins, l'élément métaphysaire (1) présente verticalement sur la totalité de sa hauteur, un trou débouchant (1c). Dans ce trou (1c) est engagée une portée cylindrique (2c) formée en bout de l'élément diaphysaire (2). L'extrémité de la portée cylindrique (2c) présente une portée filetée (2c1) destinée à recevoir un organe d'assemblage sous forme d'un écrou (3) logé dans un chambrage (1c1) formé coaxialement au trou(1c).

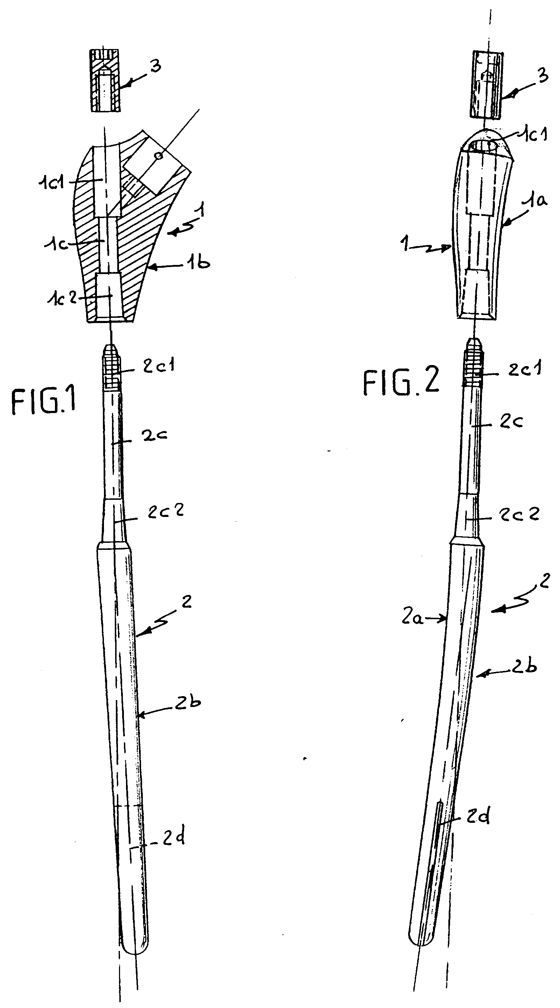

La portée cylindrique (2c), au niveau de son raccordement avec le corps de l'élément diaphysaire (2), présente une portée tronconique (2c2). Cette portée (2c2), après engagement de la tige (2c) dans le trou (1c), coopère avec une portée complémentaire (1c2) formée coaxialement audit trou (1c), à l'opposé du chambrage (1c1) (figure 1).

A noter que l'élément métaphysaire (1) est réalisé essentiellement selon deux familles différentes, pour correspondre au fémur cylindrique et au fémur conique. Chaque famille ayant notamment sa propre courbure dans le plan frontal (1b) du côté de la corticale. De même, l'élément diaphysaire (2) est réalisé sous forme d'une tige circulaire de différents diamètres et de différentes longueurs, afin d'être le plus congruent à l'os en partie distale.

On prévoit, pour des raisons de rigidité et dans le cas de longueurs de tiges importantes, de pratiquer en bout de l'élément diaphysaire, une fente (2d), afin de diminuer la rupture d'élasticité entre le métal et l'os, évitant ainsi tout remodelage osseux et risque de rupture de l'os.

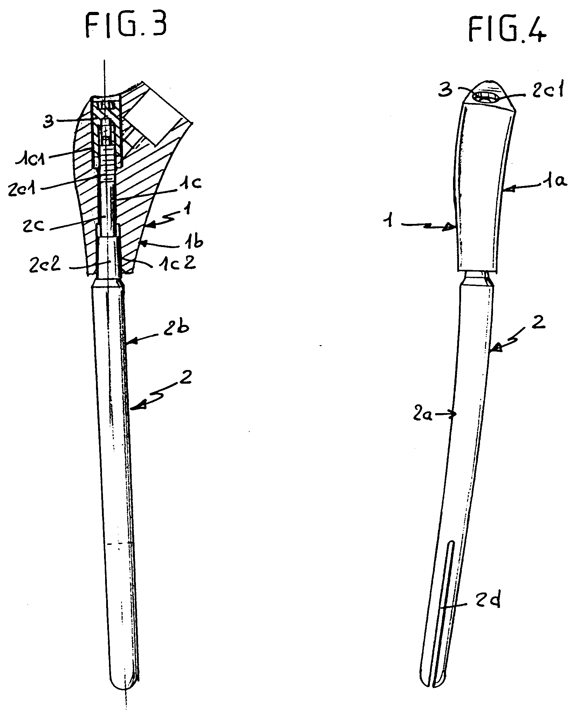

Comme il sera indiqué dans la suite de la description, avant impaction de la tige fémorale de reprise telle que définie, les éléments métaphysaire (1) et diaphysaire (2) sont assemblés au moyen de l'écrou (3), logé dans le chambrage (1c1) et vissé sur la portée filetée (2c1) après engagement de la portée (2c) dans le trou (1c), tout en permettant un mouvement relatif de rotation entre les deux éléments (1) et (2). On renvoie à la figure 3, qui montre ce préassemblage des éléments (1) et (2) avant blocage complet de l'écrou (3).

Pour la mise en place de cette tige fémorale et la préparation du canal médullaire, on utilise, un appareil de pose spécialement adapté à la conception spécifique de la tige.

Il convient d'analyser le mode opératoire pour l'impaction de cette tige fémorale de reprise.

Après planification à partir de calques, on procède au calibrage de la partie proximale et de la partie distale, en fonction de la planification retenue.

On réalise alors le râpage de la partie distale
A ce niveau, il est possible d'introduire l'ensemble de la tige fémorale de reprise dont les éléments métaphysaire (1) et diaphysaire (2) sont rendus solidaires par l'écrou (3), tout en étant libre en rotation, comme indiqué précédemment et comme montré figure 3. Les deux éléments (1) et (2), glissés à l'intérieur du canal médullaire se positionnent indiféremment dans leur bonne courbure, pour épouser automatiquement la double courbure anatomique du fémur, compte-tenu de leur montage en libre rotation.

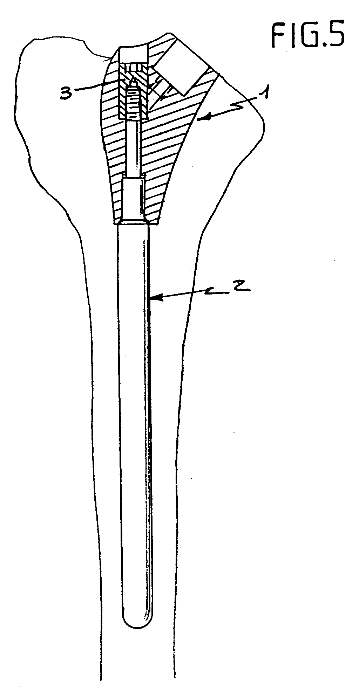

Après positionnement des deux éléments (1) et (2), le chirurgien bloque l'écrou (3) (figure 5).

Il en résulte que la combinaison du blocage et de la présence des deux courbures (1a) et (2a), de valeur et de sens différent, constitue un système autobloquant, de sorte que la partie osseuse proximale est mise en compression par rapport à la partie osseuse distale.

On prévoit de recouvrir l'élément diaphysaire d'une matière céramique dense, non ostéoconductrice mais à résorption programmable en modifiant la composition de ladite matière. En effet, la compression primaire va diminuer progressivement dans le temps, pour augmenter progressivement la tenue proximale de la tige.

De même, l'élément diaphysaire peut être équipé d'un manchon rapporté également réalisé dans une matière céramique dense, non ostéoconductrice, mais à résorbtion programmable. Ces dispositions permettent en jouant sur le diamètre du manchon, de remplir parfaitement la partie distale.

Les avantages ressortent bien de la description.

## Revendications

1. Tige fémorale de reprise pour prothèse de hanche, comprenant un élément métaphysaire (1) et un élément diaphysaire (2) en deux parties distinctes et indépendantes, avec des agencements d'accouplement, caractériseé en ce que chaque élément (1) et (2) a une courbure sagittale antéro-postérieure spécifique (1a) (2a), pour correspondre à la double courbure anatomique du fémur les agencements d'accouplement des dits éléments étant aptes, d'une part, à les rendre solidaires avec capacité de rotation libre au moment de l'introduction dans le canal médullaire pour leur auto-adaptation et, d'autre part, à les rendre solidaires en créant un effet de compression de l'élément métaphysaire (1) par rapport à l'élément diaphysaire (2), après introduction dans le canal médullaire.

2. Tige selon la revendication 1 caractérisée en ce que les agencements sont constitués par un trou débouchant (1c) formé sur la totalité de la hauteur de l'élément métaphysaire (1), pour recevoir avec capacité de rotation, une portée cylindrique (2c) formée en bout de l'élément diaphysaire (2) et dont l'extrémité (2c1) est conformée pour coopérer avec au moins un moyen d'assemblage rapporté (3) de l'élément métaphysaire (1).

3. Tige selon la revendication 2, caractérisée en ce que les moyens sont constitués par un écrou (3) logé dans un chambrage (1c1) du trou débouchant (1c) de l'élément métaphysaire (1) et coopérant avec une portée filetée (2c1) formée en bout de la portée cylindrique (2c).

4. Tige selon la revendication 2, caractérisée en ce que la portée cylindrique (2c), au niveau de son raccordement avec l'élément diaphysaire présente une portée tronconique (2c2) coopérant avec une portée complémentaire (1c2) du trou (1c) de l'élément métaphysaire (1).

5. Tige selon la revendication 1, caractérisée en ce que l'extrémité distale de l'élément diaphysaire (2) présente une fente (2d).

6. Tige selon la revendication 1, caractérisée en ce que l'élément diaphysaire (2) est recouvert directement ou par l'intermédiaire d'un élément rapporté d'une matière céramique dense, non ostéoconductrice et à résorption programmable en modifiant la composition de ladite matière.

7. Tige selon la revendication 1, caractérisée en ce que elle comprend plusieurs éléments diaphysaires (2) composés d'une tige circulaire de différents diamètres et de différentes longueurs.

## Claims

1. Repair femoral shaft for hip prosthesis, consisting of a metaphyseal element (1) and a diaphyseal element (2) in two distinct and independent parts, with coupling features, characterised in that each element (1) and (2) has a specific antero-posterior sagittal curvature (1a) (2a) to correspond with the double anatomic curvature of the femur, the coupling features of said elements being capable, on the one hand, of more firmly joining them with the capacity for free rotation at the moment of introduction into the medullary canal for their auto-adaptation, and, on the other hand, of more firmly joining them by creating a compression effect of the metaphyseal element (1) relative to the diaphyseal element (2), after introduction into the medullary canal.

2. Shaft according to claim 1, characterised in that the features consist of a through-hole (1c) formed through the entire height of the metaphyseal element (1), to receive, with the capacity of rotation, a cylindrical surface (2c) formed at the extremity of the diaphyseal element (2) and the extremity of which (2c1) is shaped to cooperate with at least an attached means of assembly (3) of the metaphyseal element (1).

3. Shaft according to claim 2, characterised in that the means consists of a nut (3), lodged in a recess (1c1) in the through-hole (1c) of the metaphyseal element (1), and co-operating with a threaded surface (2c1), formed at the end of the cylindrical surface (2c).

4. Shaft according to claim 2, characterised in that the cylindrical surface (2c), at the level of its joining to the diaphyseal element, bears a tapered surface (2c2) co-operating with a complementary surface (1c2) in the hole (1c) of the metaphyseal element (1).

5. Shaft according to claim 1, characterised in that the distal extremity of the diaphyseal element (2) bears a slot (2d).

6. Shaft according to claim 1, characterised in that the diaphyseal element (2) is covered, either directly or by the intermediary of an attached element of a dense ceramic non- osteoconductive material, the resorption of which is programmable by modifying the composition of said material.

7. Shaft according to claim 1, characterised in that it consists of several diaphyseal elements (2) composed of a circular shaft of different diameters and different lengths.

## Patentansprüche

1. Femurschaftreplantat für Hüftprothesen bestehend aus einem Metaphysenelement (1) und einem Diaphysenelement (2) in zwei unterschiedlichen und unabhängigen Teilen mit Kupplungsvorkehrungen, dadurch gekennzeichnet, daß jedes Element (1) und (2) eine spezifische anteroposteriore sagittale Krümmung (1a) (2a) aufweist, um der doppelten anatomischen Krümmung des Femurs zu entsprechen, wobei die Kupplungsvorkehrungen der besagten Elemente geeignet sind, einerseits bei unbehinderter Drehfähigkeit während des Einführens in den Markkanal zwecks Selbsteinpassung für eine feste Verbindung zu sorgen und andererseits nach dem Einführen in den Markkanal durch Erzeugung eines Druckeffekts des Metaphysenelements (1) gegenüber dem Diaphysenelement (2) eine feste Verbindung herzustellen.

2. Schaft nach Anspruch 1, dadurch gekennzeichnet, daß die Vorkehrungen aus einem auf der gesamten Höhe des Metaphysenelements (1) ausgebildeten Durchgangsloch (1c) bestehen, um eine drehfähige zylindrische Lagerfläche (2c) aufzunehmen, die am Ende des Diaphysenelements (2) ausgebildet ist und deren Endstück (2c1) dazu ausgeformt ist, um mit mindestens einem angesetzten Verbindungsmittel (3) des Metaphysenelements (1) zusammenzuwirken.

3. Schaft nach Anspruch 2, dadurch gekennzeichnet, daß die Mittel aus einer Mutter (3) bestehen, die in einer Einsenkung (1c1) des Durchgangslochs (1c) des Metaphysenelements (1) sitzt und mit einem Gewindebereich (2c1), das am Ende der zylindrischen Lagerfläche (2c) ausgebildet ist, zusammenwirkt.

4. Schaft nach Anspruch 2, dadurch gekennzeichnet, daß die zylindrische Lagerfläche (2c) im Bereich der Verbindung mit dem Diaphysenelement eine kegelstumpfartige Lagerfläche (2c2) aufweist, die mit einer ergänzenden Auflagefläche (1c2) des Loches (1c) des Metaphysenelements (1) zusammenwirkt.

5. Schaft nach Anspruch 1, dadurch gekennzeichnet, daß das distale Endstück des Diaphysenelements (2) einen Schlitz (2d) aufweist.

6. Schaft nach Anspruch 1, dadurch gekennzeichnet, daß das Diaphysenelement (2) unmittelbar oder über ein angesetztes Element mit einem dichten, nicht knochenleitenden Keramikmaterial überzogen ist, dessen Resorption durch Anderung der Materialzusammensetzung programmiert werden kann.

7. Schaft nach Anspruch 1, dadurch gekennzeichnet, daß er aus mehreren Diaphysenelementen (2) besteht, die aus einem kreisförmigen Schaftteil mit unterschiedlichem Durchmesser und unterschiedlicher Länge zusammengesetzt sind.
